# EUROPEAN PATENT APPLICATION

(11) **EP 3 889 738 A1**
(43) Date of publication of application: **06.10.2021**
(21) Application number: 20461526.4
(22) Date of filing: 04.04.2020
(51) Int. Cl.: G06F 3/01, A61B 5/00

(54) **A SYSTEM AND A METHOD FOR CALIBRATING A USER INTERFACE**

(71) Applicant: Neuroforma Sp. z o.o., 15-540 Bialystok (PL)
(72) Inventor: Pawlisz, Maciej, 84-207 Bojano (PL)
(74) Representative: Kancelaria Eupatent.pl Sp. z.o.o

(57) **Abstract**

A method for calibrating a user interface, the method comprising the steps of: selecting (201) a type of exercise to be performed by a user; accessing (202) a movements database (107) to access definitions of exercises wherein each exercise comprises related information on involved body parts of a user, which shall be detected and tracked as well as a range of motion; determining (203), based on a selected exercise definition, where at least one body part of the user shall be positioned; generating instructions (205) for the user to move and position according to selected exercise requirements; awaiting assuming by the user the position according to exercise requirements by analysing video signal captured by the camera (103).

## Description

### TECHNICAL FIELD

The present invention relates to a system and a method for calibrating a user interface. The invention is usable in particular at optimizing a camera view for gaming or physical exercise systems.

### BACKGROUND OF THE INVENTION

Virtual reality or augmented reality gaming or physical exercise systems are becoming more and more popular. In such systems, a user is instructed to perform certain movements, a camera captures the user's movements and a computer system tracks and analyses these movements.

For example, a US patent application US2018315247 discloses a virtual or augmented reality rehabilitation which includes comparing reference range of motion capabilities of a body part that mirrors or matches the target body part (e.g., from a camera or a database) to captured range of motion capabilities of the target body part. The comparison may result in an assessment, a virtual animation, a virtual component, or other output, such as to be displayed on a display or an augmented reality display.

A PCT application WO2015103359 discloses a system and a method that implement video capture technology, in combination with computer program engines and proprietary algorithms to capture, analyze and objectively score human movement, and to identify, differentially diagnose and assess the root causes of observed pathokinematic (pathological movement) patterns. In this system, the user is relatively far from the camera so that the camera may capture the complete posture as well as the surrounding mat and area.

### SUMMARY AND OBJECTS OF THE PRESENT INVENTION

There is a need to optimize a camera view in a system that tracks the user's movements to calibrate the user interface accordingly to the type of movement that is going to be performed, in order to allow tracking of movement with optimal precision.

In the present invention, the user interface is calibrated such that the camera view is optimized and adjusted to the range of movement that is performed by the user. Therefore, movements of smaller objects in a small range (such as fingers of static hand) can be viewed closer than movements of larger objects (such as a dancing person).

The invention relates to a method for calibrating a user interface, the method comprising the steps of: selecting a type of exercise to be performed by a user; accessing a movements database to access definitions of exercises wherein each exercise comprises related information on involved body parts of a user, which shall be detected and tracked as well as a range of motion; determining, based on a selected exercise definition, where at least one body part of the user shall be positioned; generating instructions for the user to move and position according to selected exercise requirements; awaiting assuming by the user the position according to exercise requirements by analysing video signal captured by the camera.

Preferably, said selection step is executed by generating and outputting a graphical user interface and receiving from the user a selection of an exercise.

Preferably, the method further comprises a step of generating the selected exercise using augmented reality such that the augmented reality objects are reachable by said user.

Preferably, prior to said step of generating instructions the method is configured to execute a step of generating in the output video signal, a representation of user's body parts outline, superimposed on images captured by a camera, where the user is expected to be positioned.

Preferably, said head shall have a predefined position as well as size in an image captured by the camera.

Preferably, said size refers to a percentage of the image area.

Preferably, said outline where the user is expected to be positioned is an reference position.

Preferably, the current position of a user is acceptable as long as it differs from said reference position within a given threshold, wherein said method executes a step of manipulating said image in order to obtain said reference position in the image.

Preferably, said instructions are visual instructions embedded in output video signal.

Preferably, said instructions are audible instructions.

Preferably, once the user has assumed the required position the method is further configured to signal this fact to the user.

Preferably, prior to said selection step the method is configured to establish the user's body proportions based on a series of test exercises aimed at measuring such body proportions.

The invention also relates to a computer program comprising program code means for performing all the steps of the computer-implemented method according as described herein when said program is run on a computer, as well to a computer readable medium storing computer-executable instructions performing all the steps of the computer-implemented method as described herein when executed on a computer.

The invention further relates to a system for calibrating a user interface wherein the system comprises: a camera; an augmented reality engine configured to generate computer objects that enhance image captured by said camera; a controller, the system being characterized in that the controller is configured to execute all steps of the method according to any of claims 1-12.

### BRIEF DESCRIPTION OF THE DRAWINGS

These and other objects of the invention presented herein are accomplished by providing a system and method for calibrating a user interface. Further details and features of the present invention, its nature and various advantages will become more apparent from the following detailed description of the preferred embodiments shown in a drawing, in which:
Fig. 1 presents a diagram of the system according to the present invention;
Fig. 2 presents a diagram of the method according to the present invention;
Figs. 3A-B present examples of user interface during calibration;
Fig. 4 presents examples of monitored movements of a user; and
Fig. 5 shows examples of an embodiment using outlines for positioning.

### NOTATION AND NOMENCLATURE

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

As utilized herein, the term "example" means serving as a non-limiting example, instance, or illustration. As utilized herein, the terms "for example" and "e.g." introduce a list of one or more non-limiting examples, instances, or illustrations.

### DESCRIPTION OF EMBODIMENTS

The present invention aims at calibrating a user interface. In particular, it targets optimizing camera view to match given exercise so that exercises involving relatively small movements may be executed closer to the camera and thereby tracked with greater precision.

The above may require positioning a user in a field of view of a camera such that at least one body part of said user covers a predefined section of said field of view so that the user is able to move and reach augmented reality objects presented to the user.

In an example embodiment, a user's face may be detected, measured and then the user may be requested to move in order to reach a certain position. Based on user's face measurements it is determined what is the users theoretical movements reach and the users may be guided to position themselves so as to be able to reach the augmented reality targets (i.e. such that the user is not positioned too far or too close).

In one embodiment, after a user has been positioned as required by the present system, a camera view may be adjusted e.g. cropped or zoomed to an area of interest focusing on the user and discarding irrelevant portions of captured images.

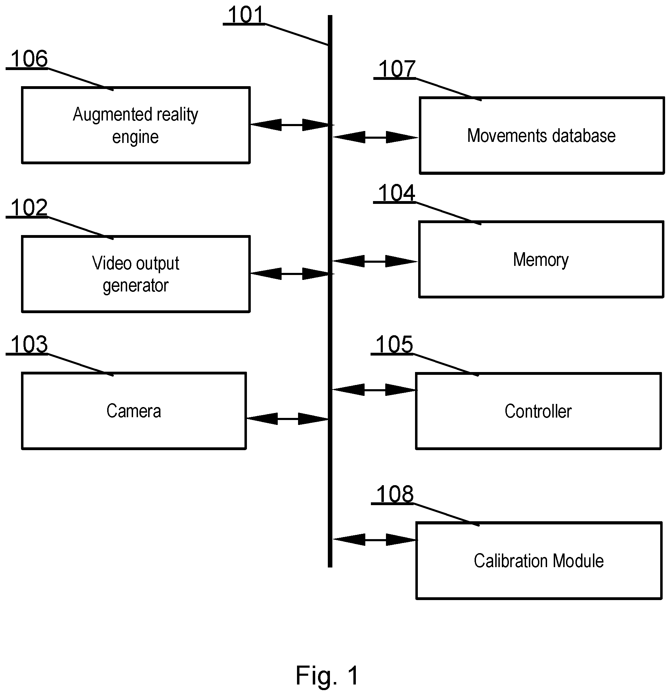

Fig. 1 presents a diagram of the system according to the present invention. The system may be realized using dedicated components or custom made FPGA or ASIC circuits. Alternatively, the system may be implemented by means of a standard computer, such as a stand-alone PC computer, a tablet, a smartphone, etc. The system comprises a data bus 101 communicatively coupled to a memory 104. Additionally, other components of the system are communicatively coupled to the system bus 101 so that they may be managed by a controller 105.

The memory 104 may store computer program or programs executed by the controller 105 in order to execute steps of the method according to the present invention.

The controller 105 receives data from a camera 103 and processes the data to obtain information on presence and positioning of users body parts in the captured image.

The system may work with any type of camera, such as a typical Internet communication camera, a higher resolution 2D camera such as an FHD or 4K camera or more sophisticated 3D cameras..

The use of said camera 103 may also allow gesture control of the complete system as known in the art. Alternatively, voice control may be employed (not shown) or other control methods known to persons skilled in the art.

A movements database 107 is configured to store definitions of movements that contain information on involved body parts of a user, which shall be detected and tracked as well as a range of motion.

The records present in the movements database 107 may define for example expected key positioning of body parts or sections of body parts e.g. a crook of an elbow, an arm, palm, an elbow, fingers, eyes, ears, feet etc. as will be recognised by a person skilled in the art.

In an alternative embodiment, such record may be defined using a series of points in space that are expected to be matched usually withing a given threshold in order to detect a matching movement.

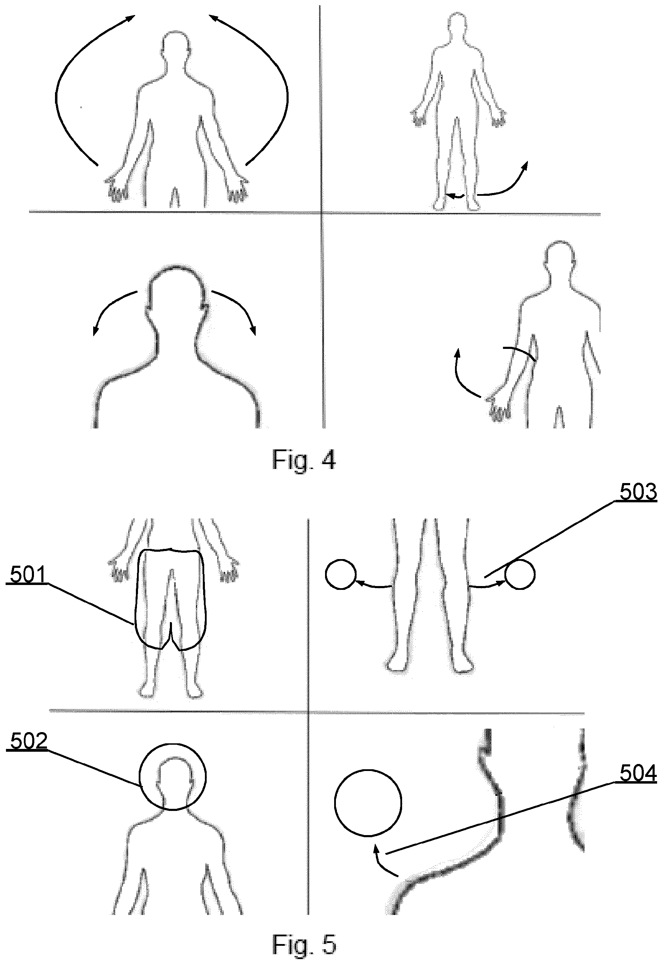

Fig. 4 presents examples of such movements. In case of the top-left scenario a user is expected to move both stretched arms up and down (i.e. virtually drawing portions of a circle). The system is to capture and identify these movements as well as match the identified movements with a definition comprised in a record of the movements database 107.

The definition of an exercise comprises information on where a given one or body parts of the user shall be positioned in the captured image. The size of the respective body parts in the image is also taken into account as typically a head (of an adult) of a given size in the image will require the user to be closer to the camera than for example in case the expected head size is much smaller.

It is clear that different persons may have different proportions of a body. In general, the further a person is from the camera, the smaller the person's head is in the image while the other body parts present in the image decrease proportionally e.g. reach of hands. In case, for example child's body proportions are set in a configuration as matching adult's proportions, bringing such child to a required distance from the camera will result in that the child's head will have an expected size in the image and therefore other body parts will have an expected size as well (in particular reach of hands).

Particular body proportions of a particular person may however be measured and stored. Such proportions may be measured physically and input to the system as configuration parameters or alternatively the system may establish such proportions based on a series of test exercises aimed at measuring the body proportions. For example, a person may be asked to stand sufficiently far from the camera to have the full body in the image and to stretch a hand as far as possible to one side at a given angle with respect to the body - then a proportion between an arm and a head may be determined.

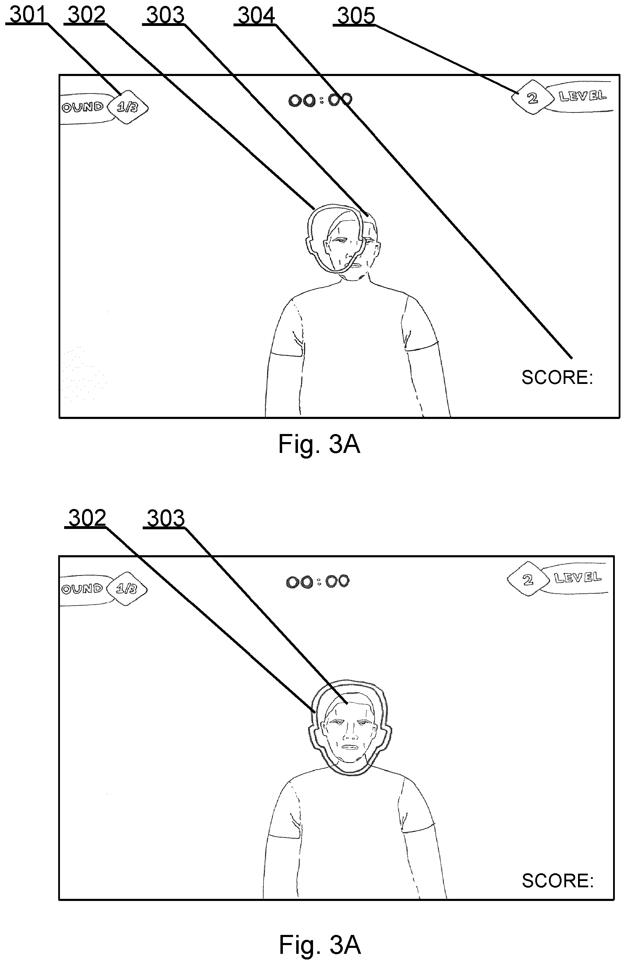

An augmented reality engine 106 is responsible for creating an interactive experience comprising camera-based images of a real-world environment where computer generated objects enhance objects present in the real world. Examples of such augmented reality images have been presented in Fig. 3A-B.

A video output generator 102 is responsible for combining the camera signal with the augmented reality graphics in order to output a signal to a suitable display.

A calibration module 108 may be configured to establish body proportions of a user as mentioned above. This module may also be responsible for positioning a person, within an area captured by the respective camera, preferably using visual or audible cues. Optionally, the calibration module 108 may also inform a user that camera positioning must be changed because a required positioning of a user is not possible at given camera setup e.g. the camera is directed too low. Positioning of said camera need not be very precise, as long as a user image may be manipulated e.g. cropped to a required positioning in the image.

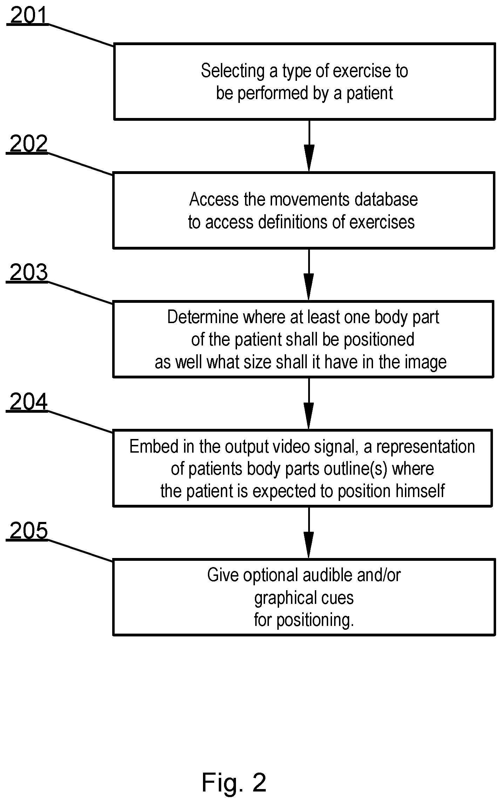

Fig. 2 presents a diagram of the method according to the present invention. The method starts at step 201 by selecting a type of exercise to perform. To this end, a graphical user interface may be presented on a display so that a user may select an exercise or a set of exercises forming a training session.

Subsequently, at step 202, the system accesses the movements database 107 to access definitions of exercises wherein each exercise comprises related information on involved body parts of a user, which shall be detected and tracked as well as a range of motion.

Next, at step 203, the process determines, based on exercise record in the movements database 107 where at least one body part, such as head, of the user shall be positioned e.g. (center, side, top, bottom) as well what size (percentage of image area) shall it have in the image (closer / farther).

Then, at step 204, the method is configured to present or otherwise embed in the output video signal, a representation of users body parts outline(s), superimposed on images captured by the camera 103, where the user is expected to be positioned (for example an ideal reference position). As explained earlier, the actual positioning of a user may differ within a given threshold as long as a user image may be manipulated e.g. cropped to a required positioning in the image.

Fig. 5 presents two use examples where an outline 501, 502 is presented during calibration and thereafter cropping is effected and the respective exercises 503, 504 are made when there is present a camera focus around the specific area of exercise.

In a more general embodiment, the user is guided by the system to assume an expected initial position which typically is within a certain threshold with respect to said ideal reference position.

Frequently, during a series of exercises there will be a need for a user to return to such expected initial position.

The guiding may be effected by audio and/or visual cues. Such ideal reference position may be defined differently for each exercise or a set of exercises. Therefore, the outline step 204 is optional.

The current positioning of a user is determined in captured images and based on that determination the system may establish what the user has to do physically in order to position within said ideal reference position.

At step 205, the user may be given instructions (displayed or audible or both) to move and position according to exercise requirements. Once the user has assumed (which is tracked by the camera image analysis) the required position this fact may be signalled to the user i.e. the exercise shall than be performed from this initial position. The aforementioned manipulation of the images e.g. cropped to a required positioning in the image, may take place at this stage.

Once properly positioned, the augmented reality engine (106) is instructed to generate augmented reality objects/targets.

Preferably such generated objects/targets are reachable by said user (i.e. without making a single step.

Alternatively, such proper positioning is a position in which a selected exercise may be correctly executed without making any movements that are not parts of the exercise.

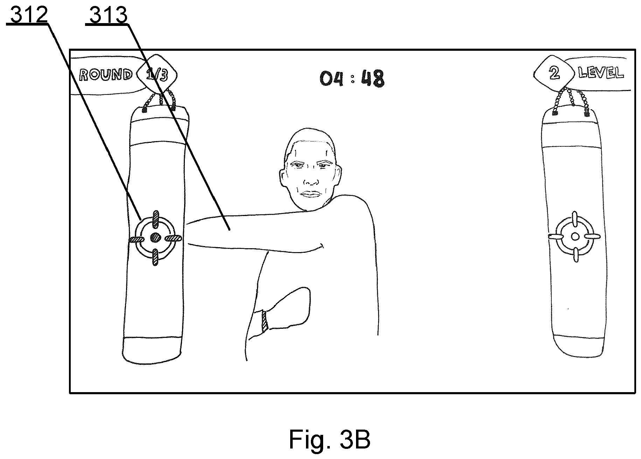

Figs. 3A, 3B present examples of augmented reality images. An image output by the present system may comprise image captured by the camera 102, with a user 303 in the image, with augmented reality features and gamification elements e.g. round identifier 301, level identifier 305 or gaming score 304.

As shown in Fig. 3A, the user may be instructed to position the user's head 303 at a particular position 302. This allows to center the user at a particular space relationship.

As shown in Fig. 3B, if the system detects that the user is too far away from the camera, the user may be instructed to move closer by providing a larger icon 302 indicating the position of the head 303.

As shown in Fig. 3C, the user may be expected to stretch a hand 313 towards particular augmented reality objects 312 (e.g. a boxing bag).

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system".

Furthermore, the present invention may take the form of a computer program product embodied in any tangible medium of expression having computer usable program code embodied in the medium.

It can be easily recognized, by one skilled in the art, that the aforementioned method for calibrating a user interface may be performed and/or controlled by one or more computer programs. Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. A method for calibrating a user interface, the method comprising the steps of:
- selecting (201) a type of exercise to be performed by a user;
- accessing (202) a movements database (107) to access definitions of exercises wherein each exercise comprises related information on involved body parts of a user, which shall be detected and tracked as well as a range of motion;
- determining (203), based on a selected exercise definition, where at least one body part of the user shall be positioned;
- generating instructions (205) for the user to move and position according to selected exercise requirements;
- awaiting assuming by the user the position according to exercise requirements by analysing video signal captured by the camera (103).

2. The method according to claim 1 wherein said selection step (201) is executed by generating and outputting a graphical user interface and receiving from the user a selection of an exercise.

3. The method according to any of previous claims, wherein the method further comprises a step of generating the selected exercise using augmented reality (106) such that the augmented reality objects are reachable by said user.

4. The method according to any of previous claims, wherein prior to said step of generating instructions (205) the method is configured to execute a step of generating (204) in the output video signal, a representation of user's body parts outline, superimposed on images captured by a camera (103), where the user is expected to be positioned.

5. The method according to claim 4 wherein said head shall have a predefined position as well as size in an image captured by the camera (103).

6. The method according to claim 5 wherein said size refers to a percentage of the image area.

7. The method according to any of previous claims, wherein said outline where the user is expected to be positioned is an reference position.

8. The method according to claim 7 wherein the current position of a user is acceptable as long as it differs from said reference position within a given threshold, wherein said method executes a step of manipulating said image in order to obtain said reference position in the image.

9. The method according to any of previous claims, wherein said instructions (205) are visual instructions embedded in output video signal.

10. The method according to any of previous claims, wherein said instructions (205) are audible instructions.

11. The method according to any of previous claims, wherein once the user has assumed the required position the method is further configured to signal this fact to the user.

12. The method according to any of previous claims, wherein prior to said selection step (201) the method is configured to establish the user's body proportions based on a series of test exercises aimed at measuring such body proportions.

13. A computer program comprising program code means for performing all the steps of the computer-implemented method according to any of claims 1-12 when said program is run on a computer.

14. A computer readable medium storing computer-executable instructions performing all the steps of the computer-implemented method according to any of claims 1-12 when executed on a computer.

15. A system for calibrating a user interface wherein the system comprises:
- a camera (103);
- an augmented reality engine (106) configured to generate computer objects that enhance image captured by said camera (103);
- a controller (105),
the system being **characterized in that**
- the controller (105) is configured to execute all steps of the method according to any of claims 1-12.
